# EUROPEAN PATENT APPLICATION

(11) **EP 3 231 873 A1**
(43) Date of publication of application: **18.10.2017**
(21) Application number: 16861381.8
(22) Date of filing: 30.08.2016
(51) Int. Cl.: C12P 21/06

(54) **OYSTER PEPTIDE CAPABLE OF ENHANCING SEXUAL FUNCTION, AND PREPARATION METHOD AND APPLICATION THEREOF**

(30) Priority: 05.11.2015 CN 201510750039
(71) Applicant: Infinitus (China) Company Ltd., Jiang Men, Guangdong 529100 (CN)
(72) Inventor: XIAO, Xiaochun, Jiangmen Guangdong 529100 (CN); ZHAI, Xufeng, Jiangmen Guangdong 529100 (CN); GUO, Xiaolei, Jiangmen Guangdong 529100 (CN); LOU, Yongjun, Jiangmen Guangdong 529100 (CN); MA, Chung Wah, Jiangmen Guangdong 529100 (CN)
(74) Representative: ABG Patentes, S.L.
(86) International application number: PCT/CN2016/097358
(87) International publication number: WO 2017/076112

(57) **Abstract**

The present invention relates to biotechnology field, disclosing a method for preparing a sexual function-improving oyster peptide and the use thereof. The method comprises: grinding oyster flesh, calcium salt and water to obtain oyster flesh pulp; enzymatic hydrolyzing oyster flesh pulp, centrifuging and collecting the supernatant to obtain an oyster enzyme-hydrolyzed raw solution; decoloring the oyster enzyme-hydrolyzed raw solution and removing impurities to obtain a fine oyster peptide solution; concentrating and spay drying the fine oyster peptide solution to obtain the oyster peptide. In the present invention, oyster flesh is pretreated with calcium salt prior to enzymatic hydrolysis to activate and releases oyster endogenous enzymes therefore reducing the use of enzymes in subsequent enzymatic hydrolysis; at the same time, cooperating with the endogenous enzymes, two steps enzymolysis is carried out to produce an oyster peptide with a strong antioxidant activity and a significant improvement in sexual function.

## Description

### CROSS REFERENCE TO RELATED APPLICATIONS

The present application is a National Phase entry of PCT Application No. PCT/CN2016/097358, filed August 30, 2016, which claims the priority of Chinese Patent Application No. 201510750039.X, as filed on November 5, 2015 and titled with "SEXUAL FUNCTION-IMPROVING OYSTER PEPTIDE, PREPARATION METHOD AND USE THEREOF", and the disclosure of which is incorporated herein by reference.

### FIELD

The present invention relates to the field of biotechnology, specifically to sexual function-improving oyster peptide and the preparation method and use thereof.

### BACKGROUND

Sexual dysfunction is a common disease in male, affecting about 10% of adult male. Along with the quick pace of modem life, pressures from work, family, finance and so on increase, so the percentage of people having sexual dysfunction also increases.

Oyster is one type of bivalve mollusks, which belongs to *Mollusca, Lamellibranchia, Ostreidae.* Oyster is the world's largest aquaculture shellfish and also one of the top four aquaculture shellfishes in China. It mainly distributes in temperate and tropical coastal areas. It was recorded in <<Ben Cao Shi Yi>> that oyster flesh has functions of "cooking, controlling deficiency, ......eating raw in the ginger vinegar, controlling erysipelas and drunk hot as well as thirst quencher". But the seasonal produce and strong fishy smell of oyster limit its use. At present, oysters are mainly sold as fresh or as dried seafood. Thus, using oysters as a material and the conjunction with biolysis technology to prepare peptide will expand its application areas and increase its added value. This has a great economic value and social significance. At present, during the preparation of oyster peptide using enzymolysis, the amount of enzyme used in the process is too much, causing the high cost of production. In the meantime, oyster peptides generated by different enzymolysis technologies are not quite the same. This is shown on their functions and the intensity of their functions. Through different enzymolysis technologies to obtain highly effective active peptide is a focus of technical personnel.

### SUMMARY

In view of the above, an object of the present invention is to provide a sexual function-improving oyster peptide and preparation method and use thereof. The preparation method described here can reduce the usage of enzyme and enhance the functions of oyster peptide on sexual function-improving and antioxidant functions. The oyster peptide in the present invention can be used in the manufacture of relative health care products, foods and pharmaceuticals.

To achieve the object of the present invention, the following technologic solution is provided.

A method for preparing the sexual function-improving oyster peptide comprises steps of:
1) grinding oyster flesh, calcium salt and water to obtain oyster flesh pulp;
2) performing enzymolysis on oyster flesh pulp, collecting supernatant after centrifuge to obtain an oyster enzyme-hydrolyzed raw solution;
3) decoloring the oyster enzyme-hydrolyzed raw solution and removing impurities to obtain a fine oyster peptide solution;
4) concentrating the fine oyster peptide solution and performing spray dehydration to obtain the oyster peptide.

To overcome the drawbacks of current enzymolysis technologies, such as weak targeting effect, high usage of enzyme and low yield of peptide, calcium salt is used to pre-treat the oyster flesh before enzymolysis in the present invention. This can activate endogenous enzymes in oyster flesh, which can hydrolyze oyster proteins efficiently and specifically, consequently, reducing the use of commercial proteases and the cost of production. Working in coordination with later enzymolysis, the step described above not only increases the protein yield, but also gives the oyster peptide a better function effect.

Herein, preferably, the calcium salt is food grade calcium chloride, calcium lactate, calcium carbonate, calcium hydrogen phosphate or calcium citrate.

Preferably, amount of calcium salt is 0.1%∼0.3% of the mass of oyster flesh.

In some embodiments of the present invention, the calcium salt is 0.1% calcium chloride, 0.3% calcium lactate or 0.2% calcium citrate.

Preferably, the water in step 1) is softened water at an amount of 0.5 to 1.0 fold of the mass of oyster flesh. In some embodiments of the present invention, the water is softened water at an amount of 0.5, 0.8 or 1.0 fold of the mass of oyster flesh.

After the pre-treatment of oysters with calcium salt, regular enzymolysis can be carried on using enzymes at a less amount.

In the present invention, preferably, step 2) is: perform enzymolysis of oyster flesh pulp by using neutral protease or alkaline protease and then by flavourzyme, inactivate enzymes after enzymolysis; centrifuge; collect the supernatant, which is the oyster enzyme-hydrolyzed raw solution.

More preferably, step 2) is: stir the oyster flesh pulp at 35°C~45°C for 1h-2h; add neutral protease or alkaline protease; heat to 50°C~60°C after adjust the pH to continue hydrolysis for 5h-8h; adjust the pH to 5.0 to 5.5; add flavourzyme to continue enzymatic hydrolysis at 50°C~60°C; inactivate the enzymes 2h-3h after the hydrolysis; collect supernatant after centrifuge. This is the oyster enzyme-hydrolyzed raw solution.

More preferably, the neutral protease is one or more of papain, bromelain, animal proteolytic enzyme and complex protease Protamex; the amount of neutral protease or alkaline protease added is 0.3 to 0.8 ‰ of the amount of oyster flesh pulp; the alkaline protease is one or more of Alcalase alkaline protease, FoodPro Alkaline Protease alkaline protease and trypsin; the amount of the flavourzyme is 0.5 to 1.0 ‰ of the mass of the oyster flesh pulp.

Herein, the pH is adjusted to 6.0 to 7.0 for enzymatic hydrolysis when using neutral protease; the pH is adjusted to 7.5 to 8.5 for enzymatic hydrolysis when using alkaline protease.

Preferably, step 3) is: add activated carbon to the oyster enzyme-hydrolyzed raw solution; stir, decolor and filter the mixture. The filtered solution is a fine oyster peptide solution.

More preferably, step 3) is: add activated carbon to the oyster enzyme-hydrolyzed raw solution; stir at 45°C∼55°C for 0.5h∼1.0h; perform filtration. The filtered solution is a fine oyster peptide solution.

By using the method in the present invention to prepare oyster peptide, the amount of neutral protease or alkaline protease consumed is less than 0.8 ‰ of the amount of oyster flesh pulp and no more than 1.0 ‰ for flavourzyme; while without calcium salt pretreatment, the same enzymatic hydrolysis process consume neutral protease or alkali protease at a amount of 1.0% of the oyster flesh pulp and 1.0% for the flavourzyme, and the amount of the enzymic preparations used is higher. At the same time, enzymatic hydrolysis is carried out in two steps in the present invention. The oyster peptide obtained by this method are more effective on enhancing the sexual function of the mouse and the antioxidant function in vitro, compared with the enzymatic hydrolysis using the same or other enzymes.

Based on the above technical effects, the present invention provides the oyster peptide prepared by the process of the present invention and the use of the oyster peptide in the preparation of the health care products, foods and pharmaceuticals for improving sexual function and/or antioxidant. Wherein the medicament and the health care product can be pills, capsules, tablets, powder, granule, oral solutions or paste.

According to the above technical scheme, oyster flesh is pretreated with calcium salt prior to enzymatic hydrolysis to activate and releases oyster endogenous enzymes, therefore reducing the use of enzymes in subsequent enzymatic hydrolysis. At the same time, cooperating with the endogenous enzymes, two-step enzymolysis is carried out to produce an oyster peptide with a strong antioxidant activity and a significant improvement in sexual function.

### DETAILED DESCRIPTION

The present invention provides a preparation method for sexual function-improving oyster peptide. One of ordinary skill in the art can use this disclosure for reference and improve the technological parameter to reach the same result. In particular, all similar replacements and changes are obvious for those skilled in the art, so they will be considered within the scope of the present invention. The method and use of the oyster peptide have been described in preferred embodiment. It is apparent that others can use the technology in the present invention through reasonable changes, modifications and combinations within the scope of the present invention.

In the embodiments, the method of detecting the antioxidant activity (reducing capacity, DPPH IC₅₀) of oyster peptide in vitro and the method for enhancing sexual function of adult male mice are described in the following steps.
1. Reducing capacity of the samples was measured at 700 nm using a potassium ferricyanide reduction system. The prepared sample was diluted at a gradient protein concentration; 2ml of the sample solution was added to the test tube and mixed with 2ml 0.2ml / L phosphate buffer (PBS, pH 6.6) and 2ml 1% (m / V) potassium ferricyanide; mixed evenly in the 50°C constant temperature water bath for 20min; 2ml 10% TCA was added and mixed thoroughly. 2ml supernatant was taken from the test tube, 2ml distilled water and 0.4ml 0.1% FeCl3 solution were added, mixed well and stood for 10min, absorbance at a wavelength of 700nm was measured. Each sample was operated in the same manner as described above and measured three times in parallel (reducing capacity IC₅₀ value is the protein concentration (mg / ml) when A700nm = 0.5).
2. Adult male mice (weight 18∼22g) were randomly divided into blank control group, sample group and comparative sample group. Each group has 12 mice. All mice were treated for 28 days continuously.

48 hours before the experiment, female rats were injected subcutaneously with estradiol benzoate (0.02 mg / mouse) to reach the same estrum. The test was carried out at 7 to 11 pm. After 30 min of the last administration, male mice were placed in the cage for 5 min to suit the environment. Then, one female was added to each cage. The following indexes were recorded:
1) interval time between the female mouse was put into the cage and the male tried to capture the female (capture latency);
2) within 20 min, the times male mouse caught the female or climbed the back of the female (number of capture);
3) within 20 min, the number of licking penis after male mouse caught the female or climbed the back of the female (ejaculation times);
4) interval time between climbing back and licking penis of male mouse (ejaculation latency).
5) percentage of capture and ejaculation within 20 min:
   Capture percentage =mouse who has capture behavior/total mice
   Ejaculation percentage=mouse who has ejaculation behavior/total mice

Data analysis: experimental data were analyzed by SPSS19.0 software. The experimental data of each group were expressed by mean value. If the variance homogeneity condition of each group was satisfied, one-way ANOVA was used to compare the variance. If not, the comparison between the groups was analyzed by Welch method, and the difference was significant with p <0.05.

The method for preparing the sexual function-improving oyster peptide in the present invention is further described below.

### Example 1: Method for Preparing the Oyster Peptide

1) The oysters were shelled; the flesh was cleaned and minced. Calcium chloride (0.1 % of the flesh mass) and water (same amount as the flesh) were added to the oyster flesh and passed colloidal mill to get oyster flesh pulp.
2) Oyster flesh pulp was stirred at 45°C for 1h. After adding papain at a amount of 0.3 ‰ of the oyster flesh pulp, the pH of the system was adjusted to 6.5; hydrolysis was carried out at 55°C for 6 hours; then the pH of the system was adjusted to 5.5 by using 0.1 mol / L HCl solution; flavourzyme (1.0 ‰ of the flesh mass) was added and hydrolysis was carried out for 2 hours; enzymes were inactivated by heating at 95°C for 15 min; finally, centrifuged and collected the supernatant. This was the oyster enzyme-hydrolyzed raw solution.
3) Activated carbon (0.6% of the solution mass) was added to oyster enzyme-hydrolyzed raw solution; stirred at 50°C for 1 hour; filtered the mixture. The filtered solution was a fine oyster peptide solution.
4) The fine oyster peptide solution was concentrated to a solution containing more than 35% of the solid and spray dried by vacuum concentration method to obtain the oyster peptide sample 1.

### Example 2: Method for Preparing the Oyster Peptide

1) The oysters were shelled; the flesh was cleaned and minced. Calcium lactate (0.3% of the flesh mass) and water (half amount as the flesh) were added to the oyster flesh and passed colloidal mill to get oyster flesh pulp.
2) Oyster flesh pulp was stirred at 35°C for 2h. After adding Alcalase at a amount of 0.8 ‰ of the oyster flesh pulp, the pH of the system was adjusted to 8.0; hydrolysis was carried out at 60°C for 5 hours; then the pH of the system was adjusted to 5.0 by using 0.1 mol / L HCl solution; flavourzyme (0.7 ‰ of the flesh mass) was added and hydrolysis was carried out for 2.5 hours; enzymes were inactivated by heating at 95°C for 15 min; finally, centrifuged and collected the supernatant. This was the oyster enzyme-hydrolyzed raw solution.
3) Activated carbon (0.3% of the solution mass) was added to oyster enzyme-hydrolyzed raw solution; stirred at 55°C for 0.5 hour; filtered the mixture. The filtered solution was a fine oyster peptide solution.
4) The fine oyster peptide solution was concentrated to a solution containing more than 45% of the solid and spray dried by vacuum concentration method to obtain the oyster peptide sample 2.

### Example 3: Method for Preparing the Oyster Peptide

1) The oysters were shelled; the flesh was cleaned and minced. Calcium citrate (0.2% of the flesh mass) and water (80% of the flesh mass) were added to the oyster flesh and passed colloidal mill to get oyster flesh pulp.
2) Oyster flesh pulp was stirred at 40°C for 1.5h. After adding papain at a amount of 0.6 ‰ of the oyster flesh pulp, the pH of the system was adjusted to 7.0; hydrolysis was carried out at 50°C for 8 hours; then the pH of the system was adjusted to 5.3 by using 0.1 mol / L HCl solution; flavourzyme (0.5 ‰ of the flesh mass) was added and hydrolysis was carried out for 3 hours; enzymes were inactivated by heating at 95°C for 15 min; finally, centrifuged and collected the supernatant. This was the oyster enzyme-hydrolyzed raw solution.
3) Activated carbon (0.5% of the solution mass) was added to oyster enzyme-hydrolyzed raw solution; stirred at 55°C for 0.7 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide sample 3.

### Example 4: Method for Preparing the Oyster Peptide

1) The oysters were shelled; the flesh was cleaned and minced. Calcium citrate (0.2% of the flesh mass) and water (80% of the flesh mass) were added to the oyster flesh and passed colloidal mill to get oyster flesh pulp.
2) Oyster flesh pulp was stirred at 40°C for 1h. After adding bromelain at a amount of 0.4‰ of the oyster flesh pulp, the pH of the system was adjusted to 6.0; hydrolysis was carried out at 52°C for 7 hours; then the pH of the system was adjusted to 5.1 by using 0.1 mol / L HCl solution; flavourzyme (0.9‰ of the flesh mass) was added and hydrolysis was carried out for 2 hours; enzymes were inactivated by heating at 95°C for 15 min; finally, centrifuged and collected the supernatant. This was the oyster enzyme-hydrolyzed raw solution.
3) Activated carbon (0.5% of the solution mass) was added to oyster enzyme-hydrolyzed raw solution; stirred at 55°C for 0.7 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide sample 4.

### Example 5: Method for Preparing the Oyster Peptide

1) The oysters were shelled; the flesh was cleaned and minced. Calcium citrate (0.2% of the flesh mass) and water (80% of the flesh mass) were added to the oyster flesh and passed colloidal mill to get oyster flesh pulp.
2) Oyster flesh pulp was stirred at 35°C for 2h. After adding animal proteolytic enzyme at a amount of 0.5‰ of the oyster flesh pulp, the pH of the system was adjusted to 6.5; hydrolysis was carried out at 50°C for 8 hours; then the pH of the system was adjusted to 5.2 by using 0.1 mol / L HCl solution; flavourzyme (0.8‰ of the flesh mass) was added and hydrolysis was carried out for 2.5 hours; enzymes were inactivated by heating at 95°C for 15 min; finally, centrifuged and collected the supernatant. This was the oyster enzyme-hydrolyzed raw solution.
3) Activated carbon (0.5% of the solution mass) was added to oyster enzyme-hydrolyzed raw solution; stirred at 55°C for 0.7 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide sample 5.

### Example 6: Method for Preparing the Oyster Peptide

1) The oysters were shelled; the flesh was cleaned and minced. Calcium citrate (0.2% of the flesh mass) and water (80% of the flesh mass) were added to the oyster flesh and passed colloidal mill to get oyster flesh pulp.
2) Oyster flesh pulp was stirred at 45°C for 1h. After adding complex protease Protamex at a amount of 0.7‰ of the oyster flesh pulp, the pH of the system was adjusted to 7.0; hydrolysis was carried out at 57°C for 6 hours; then the pH of the system was adjusted to 5.4 by using 0.1 mol / L HCl solution; flavourzyme (0.5‰ of the flesh mass) was added and hydrolysis was carried out for 3 hours; enzymes were inactivated by heating at 95°C for 15 min; finally, centrifuged and collected the supernatant. This was the oyster enzyme-hydrolyzed raw solution.
3) Activated carbon (0.5% of the solution mass) was added to oyster enzyme-hydrolyzed raw solution; stirred at 55°C for 0.7 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide sample 6.

### Example 7: Method for Preparing the Oyster Peptide

1) The oysters were shelled; the flesh was cleaned and minced. Calcium citrate (0.2% of the flesh mass) and water (80% of the flesh mass) were added to the oyster flesh and passed colloidal mill to get oyster flesh pulp.
2) Oyster flesh pulp was stirred at 38°C for 1.5h. After adding trypsin at a amount of 0.5‰ of the oyster flesh pulp, the pH of the system was adjusted to 8.5; hydrolysis was carried out at 55°C for 7 hours; then the pH of the system was adjusted to 5.5 by using 0.1 mol / L HCl solution; flavourzyme (0.6‰ of the flesh mass) was added and hydrolysis was carried out for 2 hours; enzymes were inactivated by heating at 95°C for 15 min; finally, centrifuged and collected the supernatant. This was the oyster enzyme-hydrolyzed raw solution.
3) Activated carbon (0.5% of the solution mass) was added to oyster enzyme-hydrolyzed raw solution; stirred at 55°C for 0.7 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide sample 7.

### Example 8: Method for Preparing the Oyster Peptide

1) The oysters were shelled; the flesh was cleaned and minced. Calcium citrate (0.2% of the flesh mass) and water (80% of the flesh mass) were added to the oyster flesh and passed colloidal mill to get oyster flesh pulp.
2) Oyster flesh pulp was stirred at 42°C for 2h. After adding FoodPro Alkaline Protease at a amount of 0.3‰ of the oyster flesh pulp, the pH of the system was adjusted to 7.5; hydrolysis was carried out at 53°C for 6 hours; then the pH of the system was adjusted to 5.3 by using 0.1 mol / L HCl solution; flavourzyme (0.5‰ of the flesh mass) was added and hydrolysis was carried out for 2.5 hours; enzymes were inactivated by heating at 95°C for 15 min; finally, centrifuged and collected the supernatant. This was the oyster enzyme-hydrolyzed raw solution.
3) Activated carbon (0.5% of the solution mass) was added to oyster enzyme-hydrolyzed raw solution; stirred at 55°C for 0.7 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide sample 8.

### Comparative Example 1: Method for Preparing the Comparative Oyster Peptide

1) The oysters were shelled; the flesh was cleaned and minced. Water was added at the same amount as the flesh to the oyster flesh and passed colloidal mill to get oyster flesh pulp.
2) After adding papain (1.0 ‰ of the flesh mass) and flavourzyme (1.0% of the flesh mass) to the oyster flesh pulp, the pH of the system was adjusted to 6.5; hydrolysis was carried out at 55°C for 8 hours; enzymes were inactivated by heating at 95°C for 15 min; finally, centrifuged and collected the supernatant. This was an oyster enzyme-hydrolyzed raw solution.
3) Activated carbon (0.6% of the solution mass) was added to oyster enzyme-hydrolyzed raw solution; stirred at 50°C for 1 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide comparative sample 1.

### Comparative Example 2: Method for Preparing the Comparative Oyster Peptide

1) The oysters were shelled; the flesh was cleaned and minced. Calcium chloride (0.1 % of the flesh mass) and water (80% of the flesh mass) were added to the oyster flesh and passed colloidal mill to get oyster flesh pulp.
2) Oyster flesh pulp was stirred at 40°C for 1.5h. After adding Alcalase (1 ‰ of the flesh mass) and flavourzyme (1 ‰ of the flesh mass) to the oyster flesh pulp, the pH of the system was adjusted to 8.0; hydrolysis was carried out at 50°C for 11 hours; enzymes were inactivated by heating at 95°C for 15 min; finally, centrifuged and collected the supernatant. This was the oyster enzyme-hydrolyzed raw solution.
3) Activated carbon (0.5% of the solution mass) was added to oyster enzyme-hydrolyzed raw solution; stirred at 55°C for 0.7 hour; filtered the mixture. The filtered solution was a fine oyster peptide solution.
4) The fine oyster peptide solution was concentrated to a solution containing more than 40% of the solid and spray dried by vacuum concentration method to obtain the oyster peptide comparative sample 2.

### Example 9: Recovery Rate of Enzymatic Protein and Antioxidant Activity in Vitro of Different Peptide Samples

Test samples: oyster peptides from examples 1 to 3, comparative samples from comparative examples 1 and 2. Results were shown in table 1.

**Table1. Recovery Rate of Enzymatic Protein and Antioxidant Activity in Vitro of Different Peptide Samples**

| Samples | Protein Recovery Rate (%) | Reducing Capacity* | DPPH IC₅₀ mg/ml |
|---|---|---|---|
| Oyster Peptide 1 | 79.95 | 0.52 | 3.12 |
| Oyster Peptide 2 | 82.41 | 0.56 | 3.64 |
| Oyster Peptide 3 | 80.43 | 0.51 | 3.05 |
| Comparative Sample 1 | 71.24 | 0.40 | 5.61 |
| Comparative Sample 2 | 83.01 | 0.38 | 4.99 |

| | | | |
|---|---|---|---|
| * The absorbance at A700nm when the solid matter concentration in the sample is 1 mg / ml. | | | |

From the data analysis of Table 1, it can be seen that the method for hydrolysis of oyster flesh in the present invention resulted in a high protein recovery rate (79.95-82.41%), while in comparative example 1, even a larger amount of enzyme was added, the protein recovery rate was only 71.24 %. This was mainly due to the fact that the method in the present invention activates the endogenous enzyme of the oyster flesh by adding the enzyme activator (calcium salt); hydrolysis is carried out at the optimum temperature of the endogenous enzyme (35°C~45°C) for a period of time to promote the self-dissolving of oyster flesh, thus reducing the usage amount of commercial enzymes but still get better hydrolysis efficiency. Protein recovery rate in comparative example 2 was also as high as 83.01%, verifying the importance of endogenous enzymes in hydrolysis.

At the same time, the in vitro antioxidant values (reducing capacity and DDPH clearance rate) of the oyster peptide prepared by the method of the present invention were superior to those of the comparative examples. The higher reducing capacity and lower DPPH IC₅₀ value of oyster peptide samples are mainly due to the specific enzyme combinations and two-step enzymatic hydrolysis, which are more conducive to the release of antioxidant activity.

Although comparative example 2 has a pretty high protein recovery rate, its in vitro antioxidant value (reducing capacity and DPPH) was low, whereby it was demonstrated that the enzyme combination of the present invention was an optimum enzyme combination for hydrolyzing oysters to obtain ingredients having antioxidant activity.

According to the methods in embodiments, tests were carried out on samples 4 to 8. Results showed that the protein recovery rate of samples 4 to 8 was about 82%, significantly higher than comparative sample 1 but not obviously different with comparative sample 2. The reducing capacity of samples 4 to 8 was about 0.55, higher than comparative samples 1 and 2; DDPH clearance rate of samples 4 to 8 was less than 3.5mg/ml, even lower in comparative sample 1 and 2. Taken together, the oyster peptide sample provided by the present invention has a higher reducing capacity and a lower DPPH IC₅₀ value while having a higher protein recovery rate, compared to comparative samples.

### Example 10: Effect on Male Mice Mating Ability of Different Oyster Peptide Samples

Test samples: oyster peptide from examples 1 to 3, comparative samples from comparative examples 1 and 2.

Results were shown in table 2 and 3.

**Table 2. Effect on Male Mice Mating Ability of Different Oyster Peptide Samples ( x̅ ± s , n=12)**

| Group | Dosage (g/kg) | Number of Capture | Capture Latency (s) | Capture Percentage (%) |
|---|---|---|---|---|
| Control Group | - | 5.08±1.86 | 479.50±139.03 | 75% |
| Sample 1 | 0.5 | 13.01±0.98**^{,ab} | 35.00±1.70**^{,ab} | 100% |
| Sample 2 | 0.5 | 12.33±1.47**^{,ab} | 32.00±7.39**^{,ab} | 100% |
| Sample 3 | 0.5 | 11.58±1.38**^{,ab} | 30.08±2.42**^{,ab} | 100% |
| Comparative Sample 1 | 0.5 | 6.42±0.48* | 84.42±16.91** | 85% |
| Comparative Sample 2 | 0.5 | 5.33±0.43 | 389.00±16.96 | 80% |

| | | | | |
|---|---|---|---|---|
| Note: **, p < 0.01 compared to control group; *, p < 0.05 compared to control group; a, p < 0.01 compared to comparative sample 1; b, p < 0.05 compared to comparative sample 2. | | | | |

**Table 3. Effect on Male Mice Mating Ability of Different Oyster Peptide Samples ( x̅ ± s , n=12)**

| Group | Dosage (g/kg) | Number of Ejaculation | Ejaculation Latency (s) | Ejaculation Percentage (%) |
|---|---|---|---|---|
| Control Group | - | 1.50±1.04 | 1017.33±112.18 | 25% |
| Sample 1 | 0.5 | 5.20±0.00**^{,ab} | 100.00±3.05**^{,ab} | 100% |
| Sample 2 | 0.5 | 7.08±1.10**^{,ab} | 73.75±9.36**^{,ab} | 100% |
| Sample 3 | 0.5 | 6.92±0.60**^{,ab} | 81.25±58.51**^{,ab} | 100% |
| Comparative Sample 1 | 0.5 | 3.25±0.33* | 137.17±21.74** | 90% |
| Comparative Sample 2 | 0.5 | 1.92±0.34* | 984.33±31.21** | 25% |

| | | | | |
|---|---|---|---|---|
| Note: **, p < 0.01 compared to control group; *, p < 0.05 compared to control group; a, p < 0.01 compared to comparative sample 1; b, p < 0.05 compared to comparative sample 2. | | | | |

Data analysis from table 2 and table 3 showed that: compared with the blank control group, oyster peptide samples (oyster peptide samples 1, 2, 3) have significant improvement (p < 0.01) in the number of capture and ejaculation; the oyster peptide samples (oyster peptide samples 1, 2, 3) have significant difference (p < 0.01) at capture latency and ejaculation latency compared with the blank control group and significant increase in capture rate and ejaculation rate.

Compared with comparative samples (comparative samples 1 and 2), mice administrated the oyster samples (oyster peptide samples 1, 2, 3) have a significantly higher (p < 0.01) number of capture and ejaculation and a higher capture rate and ejaculation rate.

According to the methods in embodiments, tests were carried out on samples 4 to 8. Results showed that, compared to blank control group and comparative samples, samples 4 to 8 have a significant improvement (p < 0.01) in the number of capture and ejaculation; a significant increase (p < 0.01) in the capture rate and ejaculation rate; a obvious difference (p < 0.01) on the interval time of capture latency and ejaculation latency.

At present, although the literature has not directly proved that antioxidant and sexual function has a direct relationship, the present invention found that there is a certain correlation between thess two, that is, the oyster peptide samples prepared by the present invention have a good anti-oxidation effect in vitro and also a good effect on the improvement of mice sexual function.

## Claims

1. A method for preparing a sexual function-improving oyster peptide, comprising:
1) grinding oyster flesh, calcium salt and water to obtain oyster flesh pulp;
2) enzymatic hydrolyzing oyster flesh pulp, centrifuging and collecting the supernatant to obtain an oyster enzyme-hydrolyzed raw solution;
3) decoloring the oyster enzyme-hydrolyzed raw solution and removing impurities to obtain a fine oyster peptide solution;
4) concentrating and spay drying the fine oyster peptide solution to obtain the oyster peptide.

2. The method according to claim 1, wherein the calcium salt is food grade calcium chloride, calcium lactate, calcium carbonate, calcium hydrogen phosphate or calcium citrate.

3. The method according to claim 1, wherein the amount of the calcium salt in step 1) is 0.1%~0.3% of the oyster flesh mass.

4. The method according to claim 1, wherein the step 2) comprises:
enzymatic hydrolyzing the oyster flesh pulp by neutral proteases or alkaline proteases;
adding flavourzyme for further hydrolyzation;
inactivating enzymes after enzymatic hydrolyzation;
centrifuging and collecting the supernatant to obtain an oyster enzyme-hydrolyzed raw solution.

5. The method according to claim 4, wherein the step 2) comprises:
stirring the oyster flesh pulp at 35°C~45°C for 1h~2h; adding neutral protease or alkaline protease;
adjusting the pH and heat to 50°C~60°C to continue hydrolysis for 5h-8h;
adjusting the pH to 5.0 to 5.5 and adding flavourzyme to continue hydrolysis at 50°C~60°C;
inactivating the enzymes 2h~3h after the hydrolysis;
centrifuging and collecting the supernatant to obtain an oyster enzyme-hydrolyzed raw solution.

6. The method according to claim 4, wherein the neutral protease is one or more of papain, bromelain, animal proteolytic enzyme and complex protease Protamex; the alkaline protease is one or more of Alcalase alkaline protease, FoodPro Alkaline Protease alkaline protease and trypsin.

7. The method according to claim 4, wherein the amount of the neutral protease or the alkaline protease is 0.3‰~0.8‰ of the oyster flesh pulp mass.

8. The method according to claim 4, wherein the amount of the flavourzyme is 0.5‰~1.0‰ of the oyster flesh pulp mass.

9. An oyster peptide prepared by the method according to any one of claims 1 to 8.

10. Use of the oyster peptide according to claim 9 in the manufacture of health care products, foods and pharmaceuticals for improving sexual function and/or antioxidant.
